Europäisches Patentamt

European Patent Office

Office européen des brevets

(11) Publication number: **0 171 457 B1**

# EUROPEAN PATENT SPECIFICATION

(45) Date of publication of patent specification: **25.03.92**

(51) Int. Cl.⁵: **A61K 9/24**, A61K 9/52, A01N 25/26, C05G 5/00

(21) Application number: **84109832.0**

(22) Date of filing: **17.08.84**

Divisional application 91202100.3 filed on 17/08/84.

(54) **Composition for the controlled discharge of an active ingredient, and its preparation.**

(43) Date of publication of application:
**19.02.86 Bulletin 86/08**

(45) Publication of the grant of the patent:
**25.03.92 Bulletin 92/13**

(84) Designated Contracting States:
**BE CH DE FR GB IT LI NL SE**

(56) References cited:
**EP-A- 86 093**     **EP-A- 111 144**
**EP-A- 159 604**    **AT-B- 280 478**
**AT-B- 305 498**    **GB-A- 2 025 227**
**US-A- 2 011 587**   **US-A- 3 538 214**
**US-A- 4 016 880**   **US-A- 4 060 598**

**JOURAL OF PHARMACEUTICAL SCIENCES, vol 72, no. 7, July 1983, American Pharmaceutical Association; G. KÄLLSTRAND, B. EKMAN:"Membrane-Coated Tablets: A System for the Controlled Release of Drugs", pages 772-775**

**Pharm. Ind. 44, no. 7 (1982) pgs. 735-740**

(73) Proprietor: **THE WELLCOME FOUNDATION LIMITED**
**Unicorn House 160 Euston Road**
**London NW1 2BP(GB)**

(72) Inventor: **Baker, Richard William**
**Apartment 102 505 Cypress Drive**
**Mountain View California 94303(US)**
Inventor: **Brooke, James William**
**411 Cedar Street**
**Sisters Oregon 97759(US)**
Inventor: **Smith, Kelly Lincoln**
**19295 Dayton Road**
**Bend Oregon 97701(US)**

(74) Representative: **Sandmair, Kurt, Dr. et al**
**Patentanwälte Schwabe, Sandmair, Marx**
**Stuntzstrasse 16**
**W-8000 München 80(DE)**

## Description

The present invention relates to a novel composition which affords a continuous discharge of an active ingredient in a controlled manner, to the preparation of such composition, and to its use in the medical, veterinary and other fields as it is defined below.

U.S. patents 3,845,770 and 3,916,899 relate to a composition or device comprising an active ingredient and a semipermeable membrane surrounding the active ingredient and through which is provided a passageway. In use, the composition is contacted with a fluid which permeates or diffuses through the membrane and dissolves the active ingredient. In this way, an osmotic pressure gradient is established across the membrane with the result that the solution of the active ingredient is discharged or released through the passageway into the ambient fluid. U.S. patents 4,160,452 and 4,200,098 relate to similar compositions in which a passageway or portal is also used for the discharge of the solution.

U.S. patent 4,016,880 relates to a composition or dispenser in which the semipermeable membrane or wall is provided with sites of structural weakness. As a result of the osmotic pressure gradient that builds up in use, the membrane fractures at the sites of weakness thereby forming passageways in situ, through which the solution is discharged.

J.Pharm.Sci., 1983, 72/7, pages 772 to 775 relates to tablets coated with a polyvinyl chloride membrane in which is dispersed particulate water-soluble pore-forming material. In use, an aqueous liquid dissolves the particulate water-soluble pore-forming material to give a highly porous membrane in situ. The aqueous liquid then gains access through the pores so formed to the tablet within the membrane and dissolves it, the resulting solution discharging out of the membrane through the pores.

EP-A-0 086 093 discloses a three-layered pharmaceutical film preparation which comprises one drug-storing middle layer, containing the active ingredient, and a release-controlling layer on each side of said middle layer. The layers contain plasticizers, such as polyethylene glycol, in order to make the films more flexible. When the three-layered pharmaceutical film preparations are administered to the mucosal tissue in the body cavity, they are swollen in the body liquid and expanded about 10-times. Then the body liquid permeates into the drug-storing middle layer without decomposing the release-controlling layers and dissolves gradually the drug-storing middle layer, and the drug containing therein is dissolved and then leached out of the film preparations.

EP-A-0 111 144 refers to a pharmaceutical tablet having a core and one or more enveloping sustained-release layers, the core and sustained-release enveloping layers consisting essentially of a mixture containing medicament hydrophilic gel and excipients. The release of the drug is retarded by the hydrophilic gel which swells when in contact with water. The excipients present in the tablet, for example mannitol, are used as a diluent. The excipients, e.g., mannitol, are used to provide the total weight required for the core or layers. The medicament release from the tablet is achieved either by erosion of the protective layers, or by diffusion, and the rate of release is determined by the medicament concentration as well as the hydrophilic gel and its concentration. Other factors, such as compression force in pressed tablets, appear to have little, if any, effect.

EP-A-0 159 604 which is comprised within the prior art according to Article 54(3) EPC. This document discloses a sustained-release preparation applicable to mucous membrane in oral cavity, which comprises an active ingredient and a mixture of a polymer component (A) of one or more polymers, and a polymer component (B) comprising one or more polymers, said polymer component (A) and polymer component (B) being contained in a ratio of 95 : 5 to 5 : 95 by weight, and optionally in a mixture with conventional carriers and additives. The preparation may be formed in the form of a two-layer preparation, with a layer comprising an active ingredient and for example the polymer component (A). The desired sustained-release of the active ingredient is exhibited by the use of the combination of the two-olymer components (A) and (B).

GB-A-2 025 227 discloses a pharmaceutical preparation in retard form which comprises a core containing at least one active substituent in association with the carrier or excipient, and wherein said core being provided with a semi-permeable diffusion coating comprising from 20 to 90% by weight of a water-insoluble film-former and from 10 to 80% by weight of a water-soluble polymer, e.g. polyethylene glycol. This water-soluble polymer provides the water-permeability by forming pores through which water diffuses into the tablet and drug may diffuse out.

Pharm. Ind. 44 (1982), pages 735-740, provides a similar teaching as GB-A-2 025 227 discussed above. According to the teaching of this citation pellets comprising the active ingredient (Theophyllin) are coated with ethyl cellulose films which form pores caused by addition of polyethylene glycol.

US-A-4 060 598 teaches coated tablets which are prepared by applying to a core of active material at least one layer of a coating composition made up of a film-forming aqueous synthetic resin dispersion with a resin content between 30 and 60% by weight and from 2 to 50% by weight of a water or alkaline soluble material. This coated tablet initially is air and moisture tight. After exposure to water the water-impermeable films containing the water-soluble pore-formers becomes water-permeable.

US-A-2 011 587 discloses an enteric coating for medicine intended for administration in the intestinal tract comprising a substantially water-insoluble and indigestible base composition haing incorporated therein an agent for altering the physical structure of the coating, to release the medicine in the intestines. The incorporated agent for altering the physical structure of the coating acts due to the absorption of water, the coating is then broken down. The release of the medicament is based on the priciples that under the influence of water or aqueous solutions the coating is changed in structural characteristics, to permit the release of the medicament enclosed within the coating. This structural change can be the forming of a porous coating by solving away a soluble substance incorporated in the membrane, to produce a honeycombed membrane through which the active agent may be released.

J. Pharm. Sci. 72 (1983), pages 772-775, teaches a tablet core surrounded by a porous memrane through which the gastric juices diffuse and dissolve the drug, and through which the drug diffuses out of the tablet core. This structure and principle of release is therefore very similar to the structure and release principle as disclosed in the documents discussed above, i.e. the water and drug solutions diffuse through the pores and release occurs by diffusion only.

AT-B-280 478 discloses a process for the coating of tablets containing pharmaceuticals. According to this process the tablets are coated in a per se known manner with a dispersion of one or more coating materials in a solvent. The coating material comprises a polyamide of the nylon®-type, and which is mixed with at least one mono- or disacharide.

US-A-3 538 214 teaches a pharmaceutical tablet consisting of (a) an internal matrix core comprising the medicament and (b) a coating on said core which comprises an intermediate mixture of from 0.1 to 30 parts by weight of a plastic substance and 1 part by weight of a film-modifying agent. The plastic substance is one of a low water vapor permeability, and is selected from the group consisting of cellulose acetate, ethyl cellulose, cellulose nitrate and low water-soluble polyvinyl alcohols; and the film-modifying agent is one that is readily soluble in the stomach fluids, and is selected from the group consisting of, amongst others,

sodium bicarbonate, potassium bicarbonate, lactose, etc. The film-modifying agents are used as pore-formers.

It is an object of the present invention to provide a composition which affords a continuous discharge of a solution of an active ingredient in a controlled manner.

It is also an object of the invention to provide a composition that can be manufactured simply and reproducibly without requiring any subsequent manufacturing operation, such as drilling, as is required by a number of the prior art compositions described above.

Accordingly, the present invention provides a composition which comprises a formulation containing a water-soluble active ingredient, a semipermeable membrane surrounding the formulation and a particulate water-soluble pore-forming material in the membrane, characterized in that said formulation is a prill seed containing said water-soluble active ingredient, said semipermeable membrane has a thickness of 10 to 500 $\mu$m, said pore-forming material is a monomeric pore-forming material having an average particle size from 5 to 100 $\mu$m and a diameter smaller than the thickness of the membrane and is contained in the membrane, in a form completely dispersed within the membrane, in an amount of up to 20% by weight, whereby, in use in an aqueous environment and the particle size and proportion of the pore-forming material being selected within the above ranges, the pore-forming material is dissolved forming pores in the semipermeable membrane, the actie ingredient is taken up in solution thus creating an osmotic pressure gradient across the membrane between the solution and the aqueous environment, and water from the aqueous environment is diffused through the semipermeable membrane into contact with the active ingredient concurrently while a solution of the active ingredient is discharged through the pores of the membrane into the aqueous environment, such discharge being effected substantially by osmotic pressure.

The formulation containing a water-soluble active ingredient is a prill seed, as defined hereinafter, having a coating of a water-permeable polymer, in which seed or coating or both the active ingredient is dispersed. As used herein, the prill seed of use with the polymer-coated prill seeds described above are solid particles, the largest dimension of which is from 0.1 to 4.0mm, usually from 0.2 to 0.3mm. They are normally substantially spherical in shape but may be ovoidal or even of irregular shape, and they are generally used in capsule dosage forms. Preferred examples of prill seeds include those produced from sugar such as sucrose and mannitol, starch, salt such as sodium chloride, and wax. If the active ingredient has a high water solubility, then the prill seed may have a low water solubility or indeed may even be insoluble in water. If, on the other hand, the active ingredient has a low water solubility, then it is preferred that the prill seed has a high water solubility so as to increase the osmotic pressure within the composition during its use in an aqueous environment. The preparation of such prill seeds may be carried out in accordance with standard techniques known in the art of pharmacy. Alternatively, a number of different types of prill seeds are commercially available from, for example, Ingredient Technology Corporation, Pennsauken, New Jersey, U.S.A.

The water-permeable polymer coating of use with the polymer-coated prill seeds described above may be the same polymeric material as is described hereinafter in relation to the semipermeable membrane but is, preferably, a polymeric material having a higher permeability to water. Examples of such latter material include cellulose acetate. It is also preferred that the active ingredient is dispersed within the polymeric material rather than within the prill seed. In such circumstances, from 0.5 to 5.0 parts of active ingredient on a weight basis are normally used for every one part of polymer.

The polymer-coated prill seeds may be prepared by standard techniques known in the art of pharmacy. For example, the prill seeds may be coated with a solution of the polymer optionally containing (also in solution but, if not, then in suspension) the active ingredient. Such coating is usually carried out on a fluidized bed coater in which the coating solution is sprayed into the suspending air stream thereby coating the prill seeds.

A composition, wherein the formulation is a polymer-coated prill seed, as described herein, has a number of advantages over the corresponding composition, wherein the formulation is a compressed form, for example a tablet or pill. A given dosage of the former composition, for example, has a much greater surface area than the equivalent dosage of the latter composition and this may, therefore, be of advantage in relation to active ingredients with low water solubility in that the greater surface area of the composition allows for more of the active ingredient to be dissolved and at a faster rate.

Examples of water-soluble active ingredients of use with the composition of the present invention include water-soluble active ingredients used in the fields of human and veterinary medicine and of agriculture, such as nutrients, pesticides, fungicides, herbicides, algicides, vitamins, fertilizers and soil trace minerals or elements. Particular examples of such active ingredients include d-pseudoephedrine hydrochloride, bupropion hydrochloride, soluble potassium salts such as potassium chloride, potassium citrate, potassium gluconate, chlorpheniramine maleate, propranolol hydrochloride, cimetidine, phenylpropanolamine hydrochloride, dextromethorphan hydrobromide, ascorbic acid, aspirin, acetaminophen, codeine salts, methomyl, copper sulfate and ammonium nitrate.

As used herein, a semipermeable membrane is a membrane that is permeable to water but not permeable to the active ingredient. The membrane should not be detrimental to the active ingredient and should be suitable for the use to which the composition is intended to be put. The thickness of the membrane is generally from 10 to 500$\mu$m, preferably from 25 to 50$\mu$m. The membrane may be made of any material that is suitable for use in reverse osmosis or has application in dialysis. General examples of such material include cellulose esters such as mono-, di- and triacylates including mixed esters, cellulose ethers such as ethyl cellulose, nylons, polycarbonates, poly-(dialkylsiloxanes), poly(methacrylic acid) esters, poly(acrylic acid) esters, poly(phenylene oxides), poly(vinyl alcohols), aromatic nitrogen-containing polymers, polymeric epoxides and regenerated cellulose. Specific examples include cellulose acetate, cellulose diacetate, cellulose triacetate, cellulose propionate, cellulose acetate butyrate, cellulose acetate propionate, cellulose tripropionate, ethyl cellulose and nylon® 6.

The semipermeable membrane may optionally contain one or more additives, such as a plasticizer and a water permeability-modifying agent. The plasticizer is, preferably, a non-migrating plasticizer, general examples of which include esters such as a phthalate, phosphate, citrate, adipate, tartrate, sebacate, succinate, glycolate, glycerolate, benzoate and myristate esters and sulfonamides. Specific examples include dimethyl phthalate, dipropyl phthalate, di-(2-ethylhexyl)phthalate, tributyl phosphate, triacetyl phosphate, and tributyl citrate.

As used herein, a water permeability-modifying agent is a material which is capable of enhancing the permeability of water through the semipermeable membrane. General examples of such a material include the poly(alkylene glycols), esters and polyesters of poly(alkylene glycols), polyhydric alcohols and esters and polyesters of polyhydric alcohols. Specific examples include poly(ethylene glycols) 300, 400, 600, 1500 and 1540, poly-(propylene glycol), 1,3-butyleneglycol, glycerine, ethylene glycol dipropionate and ethylene glycol butyrate.

The particulate water-soluble pore-forming material of use in the composition of the present invention, preferably, has a maximum particle size

not exceeding 500μm in its longest dimension and has an average particle size from 5 to 100μm. The material is, preferably, also insoluble in the organic solvent in which the polymeric material is dissolved for forming the semipermeable membrane as described hereinafter. Examples of water soluble pore-forming material include water-soluble sugars such as lactose, sucrose, sorbitol and mannitol, and salts such as sodium carbonate, sodium chloride, calcium chloride, potassium chloride and sodium sulphate.

The present invention also provides a process for the preparation of a composition, as defined herein, which comprises coating a prill seed formulation containing a water-soluble active ingredient with a coating mix containing in an organic solvent a solution of a material for forming a semipermeable membrane that surrounds the formulation and a suspension of particulate water-soluble pore-forming material for dispersion within the membrane, and drying the coated formulation.

The coating operation may be carried out by spraying the coating mix on to the formulation in, for example, a rotating pan coater or fluidized bed coater. The drying operation is carried out conventionally.

As mentioned previously, it is preferred that the particulate water-soluble pore-forming material is insoluble in the organic solvent. If, however, it is not, then a suspension of the material may be obtained by suspending it in a solvent in which it is insoluble, and then coating the formulation separately but simultaneously with the solution and the suspension.

Examples of an organic solvent include acetone.

As mentioned previously, the composition of the present invention may be used in the human and veterinary and other fields. In fact, it may be used in any field where there is need for controlled discharge of a water-soluble active ingredient from a composition. Thus, it is believed that the composition may be of use in the field of agriculture for, for example, the controlled discharge of water-soluble fertilizers, soil trace minerals, or elements, fungicides or herbicides. The primary application of the composition, however, is in human and veterinary medicine.

If the composition of the present invention is intended for use in human and/or veterinary medicine, then the composition and its components should, preferably, be pharmaceutically acceptable. Oral pharmaceutical compositions are preferred. Thus, the most preferred pharmaceutical compositions are capsules containing the prill seed compositions.

The present invention will now be further described with reference to drawings and examples, neither of which should be construed as limiting the invention in any way.

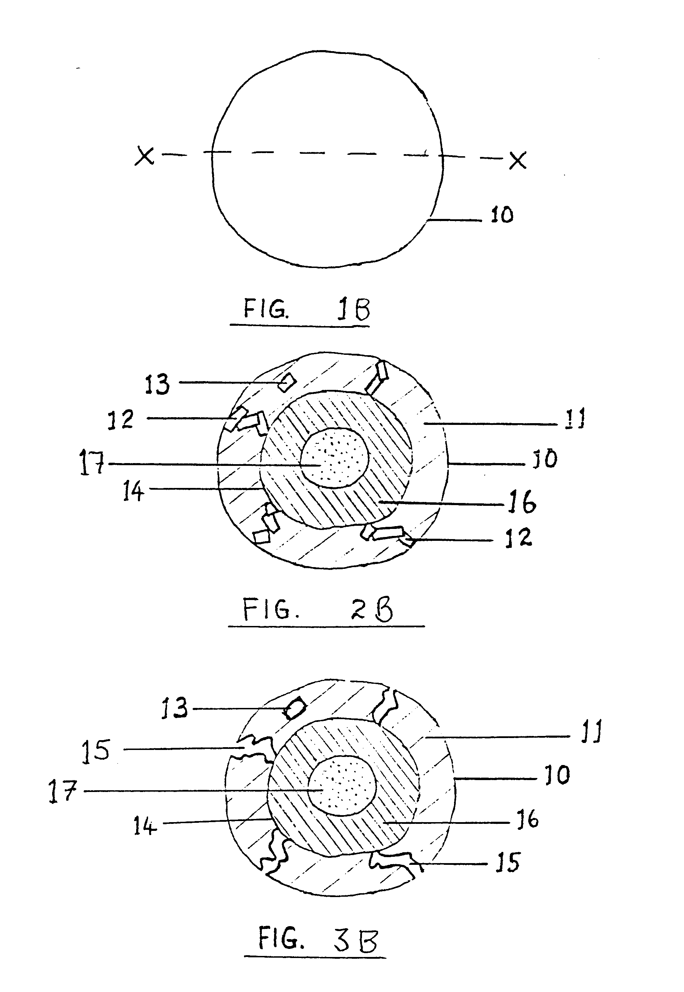

In the drawings which are not drawn to scale, Figure 1B is a plan view of a composition of the invention in which the formulation is a polymer-coated prill seed;

Figure 2B is a sectional view taken along the line X-X in a horizontal direction and illustrating the structures of the compositions before their use in an aqueous environement;

Figure 3B is the same view as depicted in Figure 2B respectively but illustrating the structures of the compositions after the pore-forming material has been dissolved.

Referring to the Figures, there are shown compositions 10 comprising a polymer-coated prill seed 14, the polymer-coating 16 containing a water-soluble active ingredient, a semipermeable membrane 11, and particulate water-soluble pore-forming material 12 and 13 dispersed within the membrane 11. In the majority of places, the pore-forming material 12 is aggregated together across the thickness of the membrane 11 whilst in a few places the material 13 is not so aggregated.

In use of the composition 10 in an aqueous environment (not shown), the pore-forming material 12 that is aggregated together across the thickness of the membrane 11 is dissolved in water from the aqueous environment thus forming pores 15 in the membrane 11. Some of the water-soluble active ingredient within the polymer coating in the immediate vicinity of the pores 15 then comes into contact with water and is taken up in solution thus setting up an osmotic pressure gradient across the membrane 11 between the aqueous solution of the active ingredient and the aqueous environment. The effect of the pressure gradient so created is that water from the aqueous environment diffuses or permeates through the membrane 11 into contact with the active ingredient while an aqueous solution of the active ingredient is discharged through the pores 15 of the membrane 11 into the aqueous environment and this process continues until the concentration of the solution of the active ingredient within the membrane 11 is substantially the same as that outside the membrane 11 in the aqueous environment at which point there is no longer any osmotic pressure gradient between the two solutions, the bulk of the active ingredient having been discharged as an aqueous solution from the composition 10 into the aqueous environment.

EXAMPLE 1

Tablets containing 100mg bupropion hydrochloride and 500mg lactose were prepared using a conventional tablet press. Fifty tablets were placed in a miniature pan coater. A polymer solution was prepared by dissolving cellulose acetate (CA 383-40 from Eastman Chemical Products, Inc., Kingsport, Tenn.) and poly(ethylene glycol) (Polyglycol® E-400 from Dow Chemical Co., Midland, Mich.) in acetone and adding impalpable lactose (particle size: 2-20um) to give a mixture containing cellulose acetate: poly(ethylene glycol): lactose in the weight % ratio of 40:40:20 and a total solids content of 50g/L. The polymer mixture was sprayed onto the tablets in the pan coater to give membrane-coated tablets, the membrane coating weighing 27mg each when dried.

Drug release rates were determined for the tablet compositions by placing them in simulated gastric buffer (pH 1.5) at 37°C and periodically measuring the bupropion hydrochloride concentration in the buffer. After 2 hr., about 45% of the bupropion hydrochloride was released; after 4 hr., about 70%; and after 6 hr., about 90%.

## EXAMPLE 2

Tablets containing 100mg bupropion hydrochloride and 500mg lactose were prepared using a conventional tablet press. Fifty tablets were placed in a miniature pan coater. A polymer solution was prepared by dissolving cellulose acetate (CA 383-40) and poly(ethylene glycol) (Polyglycol® E-400) in acetone and adding impalpable lactose to give a mixture containing cellulose acetate: poly(ethylene glycol): lactose in the weight % ratio of 67:13:20 and a total solids content of 50g/L. The polymer mixture was sprayed onto the tablets in the pan coater to give membrane-coated tablets, the membrane coating weighing 35mg each when dried.

Drug release rates were determined for the tablet compositions by placing them in simulated gastric buffer (ph 1.5) at 37°C and periodically measuring the buproprion hydrochloride concentration of the buffer. After 2 hr., about 10% of the bupropion hydrochloride was released; after 4 hr., about 25%; after 6 hr., about 40%; and after 8 hr., about 55%.

## EXAMPLE 3

Tablets containing 120g d-pseudoephedrine hydrochloride, 5mg triprolidine hydrochloride, 125mg lactose, and 28mg starch were prepared using a conventional tablet press. Fifty tablets were placed in a miniature pan coater. A polymer solution was prepared by dissolving cellulose acetate (CA 398-10 from Eastman Chemical Products, Inc., Kingsport, Tenn.) and poly(ethylene glycol) (Polyglycol® E-400) in acetone and adding powdered sodium carbonate (particle size: 30-200um) to give a mixture containing cellulose acetate: poly-

(ethylene glycol): sodium carbonate in the weight % ratio of 4:40:20 and a total solids content of 50g/L. The polymer mixture was sprayed onto the tablets in the pan coater to give membrane-coated tablets, the membrane coating weighing 64mg each when dried.

Drug release rates were determined for the tablet compositions by placing them in simulated gastric buffer (pH 1.5) and 37°C and periodically measuring the drug concentration. After 1 hr., about 33% of the d-pseudoephedrine hydrochloride and 32% of the triprolidine hydrochloride was released; after 2 hr., about 53% of each drug was released; after 3 hr., about 71% and 74%, respectively; and after 4 hr., about 97% and 85%, respectively.

## EXAMPLE 4

A total of 40g of sucrose/starch prill seeds (Nu-Pareil 20/25 mesh prills from Ingredient Technology Corp., Pennsauken, N.J.) was placed in a cylindrical bed (16 in. long x 1.5 in. diameter) and fluidized with dry compressed air at 2,0685 bar (30 psi). The prills were then coated with a solution of 12g of cellulose acetate (CA 398-10 from Eastman Chemical Products, Inc., Kingsport, Tenn.) and d-pseudoephedrine hydrochloride (36g) in ethanol (200ml) and dichloromethane (400ml) using an air brush at 30 psi. Agglomeration was avoided by intermittent application with partial drying. The polymer-coated prills were removed from the bed and allowed to dry for two hours. They were then returned to the fluidized bed and coated with a solution of 7g of cellulose acetate (CA 398-10) and 1g of poly(ethylene glycol) (Polyglycol® E-400 from Dow Chemical Co., Midland, Michigan) in 240ml of acetone containing 2g of powdered sodium carbonate (particle size: 30-200um) in suspension. The prill seed compositions thus formed were then allowed to dry.

Drug release rates were determined for the prill seed compositions by placing them in sodium chloride solutions of different concentrations at 37°C to vary the osmotic pressure driving force for drug release, and periodically measuring the d-pseudoephedrine hydrochloride concentration. When the sodium chloride concentration was 0%, the drug release rate was 90mg/hr.; when it was 5%, the release rate was 75mg/hr.; when it was 10%, the release rate was 45mg/hr.; and when it was 20%, the release rate was 10mg/hr.

## EXAMPLE 5

Sucrose seed prills (Nu-Pareil) were coated with an aspirin® (75%)-CA-398-10 (25%) mixture essentially following the procedure of Example 4 but using acetone (containing 10% by weight of total solids) instead of ethanol-dichloromethane. The polymer-coated prill seeds were dried and then overcoated with a 40:40:20 mixture of cellulose acetate (C-398-40 from Eastman Chemical Products, Inc., Kingsport, Tenn.); Polyglycol® E-400: impalpable lactose (particle size: 5-20um) in acetone (5% by weight total solids). The prill seed compositions thus formed were then allowed to dry.

Drug release rates were determined as in Example 4. After 2 hr. about 60% of the aspirin® had been released; after 4 hr. about 80% had been released; and after 8 hr. about 90% had been released.

## Claims

1. A composition which comprises a formulation containing a water-soluble active ingredient, a semipermeable membrane surrounding the formulation and a particulate water-soluble pore-forming material in the membrane, characterized in that said formulation is a prill seed containing said water-soluble active ingredient, said semipermeable membrane has a thickness of 10 to 500 $\mu$m, said pore-forming material is a monomeric pore-forming material having an average particle size from 5 to 100 $\mu$m and a diameter smaller than the thickness of the membrane and is contained in the membrane, in a form completely dispersed within the membrane, in an amount of 20 % by weight, whereby, in use in an aqueous environment and the particle size and proportion of the pore-forming material being selected within the above ranges, the pore-forming material is dissolved forming pores in the semipermeable membrane, the active ingredient is taken up in solution thus creating an osmotic pressure gradient across the membrane between the solution and the aqueous environment, and water from the aqueous environment is diffused through the semipermeable membrane into contact with the active ingredient concurrently while a solution of the active ingredient is discharged through the pores of the membrane into the aqueous environment, such discharge being effected substantially by osmotic pressure.

2. A composition according to claim 1 wherein the prill seed has a coating of a water-permeable polymer, in which seed or coating or both the active ingredient is dispersed.

3. A composition according to claim 2 wherein the water-permeable polymer is cellulose acetate.

4. A composition according to any one of the preceding claims wherein the active ingredient is effective in the field of human or veterinary medicine or in the field of agriculture.

5. A composition according to any of the preceding claims wherein the semipermeable membrane is made from a cellulose ester, a cellulose ether, nylon, a polycarbonate, a poly(dialkylsiloxane), a poly(methacrylic acid) ester, a poly(acrylic acid) ester, a poly(phenylene oxide), a poly(vinyl alcohol), an aromatic nitrogen-containing polymer, a polymeric epoxide or regenerated cellulose.

6. A composition according to any of the preceding claims wherein the particulate monomeric water-soluble pore-forming material is made from a water-soluble sugar or a salt, preferably made from lactose, sucrose, sorbitol, mannitol, sodium carbonate, sodium chloride, calcium chloride, potassium chloride and sodium sulfate.

7. A process for preparing a composition according to any of the preceding claims said process comprising coating a prill seed formulation containing a water-soluble active ingredient with a coating mix containing, in an organic solvent, a solution of a material for forming a semipermeable membrane that surrounds the prill seed and a suspension of a particulate monomeric water-soluble pore-forming material for dispersion within the membrane, said particulate material having an average particle size from 5 to 100 $\mu$m, and drying the coated formulation.

8. A pharmaceutical composition which comprises a composition according to any of claims 1 to 3, 5 and 6 in which the active ingredient is an effective ingredient in the field of human or veterinary medicine, all other components being pharmaceutically acceptable.

9. A capsule dosage form containing a pharmaceutical composition according to claim 8 wherein the formulation is a prill seed having a coating of a water-permeable polymer, in which seed or coating or both the active ingredient is dispersed.

## Revendications

1. Composition qui comprend une préparation contenant un constituant actif hydrosoluble, une membrane semi-perméable entourant la préparation et une matière particulaire hydrosoluble formant des pores dans la membrane, caractérisée en ce que la préparation est un germe granulaire contenant le constituant actif hydrosoluble, la membrane semi-perméable à une épaisseur de 10 à 500 μm, la matière formant des pores est une matière monomère formant des pores ayant une granulométrie moyenne de 5 à 100 μm et un diamètre inférieur à l'épaisseur de la membrane et est contenue dans la membrane, sous une forme complètement dispersée dans la membrane, en une quantité s'élevant de 20% en poids, de façon qu'à l'utilisation dans un milieu aqueux et la granulométrie et la proportion de la matière formant des pores étant sélectionnées dans les intervalles ci-dessus, la matière formant des pores se dissolve en formant des pores dans la membrane semi-perméable, le constituant actif passe en solution créant ainsi un gradient de pression osmotique de part et d'autre de la membrane entre la solution et le milieu aqueux, et l'eau du milieu aqueux parvient par diffusion à travers la membrane semi-perméable en contact avec le constituant actif cependant qu'une solution du constituant actif est libérée par les pores de la membrane dans le milieu aqueux, ce dégagement étant effectué en substance par la pression osmotique.

2. Composition suivant la revendication 1, dans laquelle le germe granulaire porte un enrobage d'un polymère perméable à l'eau, le constituant actif étant dispersé dans le germe ou l'enrobage ou les deux.

3. Composition suivant la revendication 2, dans laquelle le polymère perméable à l'eau et l'acétate de cellulose.

4. Composition suivant l'une quelconque des revendications précédentes, dans laquelle le constituant actif est efficace dans le domaine de la médecine humaine ou vétérinaire ou dans le domaine de l'agriculture.

5. Composition suivant l'une quelconque des revendications précédentes, dans laquelle la membrane semi-perméable est faite d'un ester cellulosique, d'un éther cellulosique, de Nylon, d'un polycarbonate, d'un poly-(dialcoylsiloxane), d'un ester de poly(acide méthacrylique), d'un ester de poly(acide acrylique), d'un poly(oxyde de phénylène), d'un poly(alcool vinylique), d'un polymère aromatique contenant de l'azote, d'un époxyde polymère ou de cellulose régénérée.

6. Composition suivant l'une quelconque des revendications précédentes, dans laquelle la matière particulaire monomère hydrosoluble formant des pores est faite d'un sucre ou sel hydrosoluble, de préférence faite de lactose, de saccharose, de sorbitol, de mannitol, de carbonate de sodium, de chlorure de sodium, de chlorure de calcium, de chlorure de potassium ou de sulfate de sodium.

7. Procédé de préparation d'une composition suivant l'une quelconque des revendications précédentes, lequel procédé comprend l'enrobage d'une préparation en germe granulaire contenant un constituant actif hydrosoluble au moyen d'un mélange d'enrobage contenant, dans un solvant organique, une solution d'une matière pour former une membrane semi-perméable qui entoure le germe granulaire et une suspension d'une matière particulaire monomère hydrosoluble formant des pores pour la dispersion dans la membrane, la matière particulaire ayant une granulométrie moyenne de 5 à 100 μm, et le séchage de la préparation enrobée.

8. Composition pharmaceutique, qui comprend une composition suivant l'une quelconque des revendications 1 à 3, 5 et 6 dans laquelle le constituant actif est un constituant efficace dans le domaine de la médecine humaine ou vétérinaire, tous les autres constituants étant pharmaceutiquement acceptables.

9. Forme dosée en capsule contenant une composition pharmaceutique suivant la revendication 1, dans laquelle la préparation est un germe granulaire portant un enrobage d'un polymère perméable à l'eau, le constituant actif étant dispersé dans le germe ou l'enrobage ou les deux.

**Patentansprüche**

1. Zusammensetzung, welche eine Formulierung, die einen wasserlöslichen aktiven Bestandteil enthält, eine semipermeable Membran, die die Formulierung umgibt, und ein teilchenartiges, wasserlösliches, Poren ausbildendes Material in der Memban umfaßt, **dadurch gekennzeichnet**, daß die Formulierung ein Sprühkristallkeim ist, der den wasserlöslichen aktiven Bestandteil enthält, daß die semipermeable Membran eine Dicke von 10 bis 500 μm aufweist, daß das Poren ausbildende Material ein monomeres, Poren ausbildendes Material mit einer mittleren Teilchengröße von 5 bis 100 μm und einem Durchmesser ist, der kleiner ist als die Dicke der Membran, und in der Membran in einer vollständig innerhalb der Membran dispergierten Form in einer Menge von 20 Gew.-% enthalten ist, wobei bei Verwendung in wässriger Umgebung und dann, wenn die Teilchengröße und der Anteil des Poren ausbildenden Materials in den oben angegebenen Bereichen ausgewählt sind, das Poren ausbildende Material unter Ausbildung von Poren in der semipermeablen Membran gelöst wird, der aktive Wirkstoff in Lösung aufgenommen und dadurch im Bereich der Membran zwischen der Lösung und der wässrigen Umgebung ein Gradient des osmotischen Druckes gebildet wird, und Wasser aus der wässrigen Umgebung durch die semipermeable Membran diffundiert und gleichzeitig in Kontakt mit dem aktiven Bestandteil gebracht wird, während eine Lösung des aktiven Bestandteils durch die Poren der Membran in die wässrige Umgebung freigesetzt wird, wobei die Freisetzung im wesentlichen durch osmotischen Druck bewirkt wird.

2. Zusammensetzung nach Anspruch 1, worin der Sprühkristallkeim einen Überzug aus einem wasserdurchlässigen Polymer hat, wobei der aktive Bestandteil in dem Keim oder dem Überzug oder in beiden dispergiert ist.

3. Zusammensetzung nach Anspruch 2, worin das wasserdurchlässige Polymer Celluloseacetat ist.

4. Zusammensetzung nach irgendeinem der vorangehenden Ansprüche, worin der aktive Bestandteil im Bereich der Human- oder Veterinärmedizin oder im Bereich der Landwirtschaft wirksam ist.

5. Zusammensetzung nach irgendeinem der vorangehenden Ansprüche, worin die semipermeable Membran aus einem Celluloseester, einem Celluloseether, Nylon, einem Polycarbonat, einem Polydialkylsiloxan, einen Polymethacrylsäureester, einem Polyacrylsäureester, einem Polyphenylenoxid, einem Polyvinylalkohol, einem aromatischen, Stickstoff enthaltenden Polymer, einem polymeren Epoxid oder aus regenerierter Cellulose hergestellt ist.

6. Zusammensetzung nach irgendeinem der vorangehenden Ansprüche, worin das teilchenartige monomere, in Wasser lösliche, Poren ausbildende Material hergestellt ist aus einem wasserlöslichen Zucker oder einem Salz, vorzugsweise hegestellt ist aus Lactose, Sucrose, Sorbit(ol), Mannit(ol), Natriumcarbonat, Natriumchlorid, Calciumchlorid, Kaliumchlorid und Natriumsulfat.

7. Verfahren zur Herstellung einer Zusammensetzung nach irgendeinem der vorangehenden Ansprüche, wobei das Verfahren das Beschichten einer Sprühkristallkeim-Formulierung, welche einen wasserlöslichen aktiven Bestandteil enthält, mit einer Beschichtungsmischung, die eine Lösung eines Materials zur Ausbildung einer semipermeablen Membran, die den Sprühkristallkeim umgibt, in einem organischen Lösungsmittel sowie eine Suspension eines teilchenartigen monomeren, wasserlöslichen, Poren ausbildenden Materials zur Dispergierung innerhalb der Membran enthält, wobei das teilchenartige Material eine mittlere Teilchengröße von 5 bis 100 μm aufweist, sowie Trocknen der beschichteten Formulierung umfaßt.

8. Pharmazeutische Zusammensetzung, welche eine Zusammensetzung gemäß irgendeinem der Ansprüche 1 bis 3, 5 und 6 umfaßt, in der der aktive Bestandteil ein im Bereich der Human- oder Veterinärmedizin wirksamer Bestandteil ist, und wobei alle weiteren Komponenten pharmazeutisch annehmbar sind.

9. Dosierungsform in Form einer Kapsel, welche eine pharmazeutische Zusammensetzung gemäß Anspruch 8 enthält, worin die Formulierung ein Sprühkristallkeim mit einem Überzug aus einem wasserdurchlässigen Polymer ist, wobei der aktive Bestandteil in dem Kristallkeim oder dem Überzug oder in beiden dispergiert ist.

X — · — · — · — · — · — · — X

10

FIG.   1 B

13
12
17
14
11
10
16
12

FIG.   2 B

13
15
17
14
11
10
16
15

FIG.   3 B